# EUROPÄISCHE PATENTSCHRIFT

(11) **EP 3 662 262 B1**
(45) Veröffentlichungstag und Bekanntmachung des Hinweises auf die Patenterteilung: **15.11.2023**
(21) Anmeldenummer: 18750410.5
(22) Anmeldetag: 02.08.2018
(51) Int. Cl.: G01N 21/3504, G01N 21/65

(54) **MUSTERBASIERTE GASQUALITÄTSÜBERWACHUNG MITTELS SPEKTROSKOPIE**
PATTERN BASED GAS QUALITY MONITORING USING SPECTROSCOPY
SURVEILLANCE DE LA QUALITÉ DU GAZ BASÉ SUR UN MOTIF PAR SPECTROSCOPIE

(30) Priorität: 04.08.2017 DE 102017117709
(43) Veröffentlichungstag der Anmeldung: 10.06.2020
(73) Patentinhaber: Carl Zeiss Jena GmbH, 07745 Jena (DE); Carl Zeiss Spectroscopy GmbH, 07745 Jena (DE)
(72) Erfinder: RODE, Michael, 07743 Jena (DE); GATTO, Alexandre, 51429 Bergisch Gladbach (DE); DEPARNAY, Arnaud, 99084 Erfurt (DE); HELGERT, Michael, 07743 Jena (DE)
(74) Vertreter: Schwarz, Markku
(86) Internationale Anmeldenummer: PCT/EP2018/071047
(87) Internationale Veröffentlichungsnummer: WO 2019/025564

(56) Entgegenhaltungen:
- US-A- 3 768 908
- US-A- 5 866 430
- US-A1- 2005 247 868
- US-A1- 2005 280 814
- US-A1- 2011 208 462
- ROYSTON GOODACRE: "Explanatory analysis of spectroscopic data using machine learning of simple, interpretable rules", VIBRATIONAL SPECTROSCOPY., Bd. 32, Nr. 1, 1. August 2003 (2003-08-01) , Seiten 33-45, XP055514795, NL ISSN: 0924-2031, DOI: 10.1016/S0924-2031(03)00045-6

## Beschreibung

Die vorliegende Anmeldung betrifft ein Verfahren und Vorrichtungen zur spektroskopischen Gasqualitätsüberwachung.

In verschiedenen Bereichen besteht ein Interesse an einer Überwachung von Gasen im Hinblick auf ihre Zusammensetzung. So kann beispielsweise durch Überwachung von Luft im Freien oder in geschlossenen Räumen eine durch Umweltverschmutzung verursachte Verschlechterung der Luftqualität erfasst werden. Eine Verschlechterung der Luftqualität kann aber unabhängig von Umweltverschmutzungseinflüssen beispielweise auch im Innenraum eines Flugzeugs auftreten, wenn bei der Gewinnung von Druckluft zur Belüftung des Innenraums aus Zapfluft von Triebwerken eine Verunreinigung eingebracht wird. Weiterhin besteht auch bei Klimatisierungs- oder Belüftungsanlagen die Gefahr einer Verunreinigung der Luft durch in der Anlage verwendete Stoffe, z.B. Öl oder Kühlflüssigkeit, oder durch in die Anlage eingebrachte Fremdstoffe.

Bestehende Systeme zur Luftqualitätsüberwachung basieren typischerweise auf einer Analyse der Luft im Hinblick auf bestimmte Schadstoffe, z.B. Ozon, CO₂, CO, Schwefeloxide, Stickoxide oder Feinstaub. Die WO 2014/134521 A1 beschreibt beispielsweise ein System, bei welchem die Konzentrationen verschiedener Zielschadstoffe erfasst und ausgewertet werden. Die für die Messung von Konzentrationen einzelner Schadstoffe erforderliche Analytik ist jedoch komplex und typischerweise empfindlich gegenüber variablen Umgebungsbedingungen. Weiterhin könnten auch unerwartete Schadstoffe auftreten, auf deren Erfassung die Analytik nicht ausgelegt ist. Die US 2005/247868 A1 beschreibt Verfahren zur Erzeugung eines biologischen Alarms, bei welchem das Vorhandensein oder die Konzentration von luftbasierten Gefahrstoffen überwacht wird.

Es ist daher eine Aufgabe der vorliegenden Erfindung, Techniken bereitzustellen, welche eine effiziente Gasqualitätsüberwachung ermöglichen.

Hierzu werden ein Verfahren zur Gasqualitätsüberwachung nach Anspruch 1 sowie eine Vorrichtung zur Gasqualitätsüberwachung nach Anspruch 11 bereitgestellt. Die Unteransprüche definieren weitere Ausführungsformen.

Gemäß einem Ausführungsbeispiel der vorliegenden Erfindung wird somit ein Verfahren zur Gasqualitätsüberwachung bereitgestellt. Das Verfahren umfasst eine spektroskopische Untersuchung einer Gasprobe aus einem zu überwachenden Raum. Die spektroskopische Untersuchung kann insbesondere durch Raman-Spektroskopie erfolgen. Die spektroskopische Untersuchung liefert ein sich über einen Wellenlängenbereich erstreckendes Messspektrum. Weiterhin wird eine Abweichung des Messspektrums von wenigstens einem Vergleichsmuster erfasst. Abhängig von der erfassten Abweichung wird eine Gasqualitätswarnung erzeugt. Insbesondere wird die Gasqualitätswarnung erzeugt, wenn eine Abweichung des Messspektrums von dem Vergleichsmuster bzw. einem der Vergleichsmuster auftritt oder die erfasste Abweichung eine bestimmte Mindeststärke aufweist. Das Vergleichsmuster kann gemeinsame Eigenschaften von Messspektren beschreiben, welche als unauffällig eingestuft werden. Das wenigstens eine Vergleichsmuster kann somit auf Basis von Messspektren erzeugt sein, welche durch Untersuchung von als unauffällig eingestuften Gasproben gewonnen wurden. Bei einer Abweichung von den durch das Vergleichsmuster beschriebenen Eigenschaften kann das durch die spektroskopische Untersuchung gewonnene Messspektrum als auffällig eingestuft und die Gasqualitätswarnung erzeugt werden. Die Gasqualitätswarnung kann somit auf effiziente Weise erzeugt werden, ohne dass eine Zusammensetzung oder einzelne Inhaltsstoffe der Gasprobe identifiziert werden müssen.

Gemäß einem Ausführungsbeispiel wird weiterhin wenigstens ein Umgebungsparameter des zu überwachenden Raums erfasst. Das wenigstens eine Vergleichsmuster kann dann abhängig von dem erfassten wenigstens einen Umgebungsparameter ausgewählt werden. Der wenigstens eine Umgebungsparameter kann eine Temperatur, einen Druck und/oder einen Feuchtigkeitsgehalt in dem zu überwachenden Raum umfassen. Durch die Auswahl des wenigstens einen Vergleichsmusters abhängig von dem wenigstens einen Umgebungsparameter kann auf effiziente Weise variablen Umgebungsbedingungen Rechnung getragen werden. Insbesondere können für verschiedene Umgebungsbedingungen jeweils passende Vergleichsmuster zum Einsatz kommen.

Gemäß einem Beispiel, welches für sich genommen nicht unter die Ansprüche fällt, wird die Abweichung durch einen Vergleich einer wellenlängenabhängigen Intensität des Messspektrums mit wenigstens einer von dem Vergleichsmuster definierten Vergleichsintensität erfasst. So kann das wenigstens eine Vergleichsmuster beispielsweise eine wellenlängenabhängige Vergleichsintensität definieren, deren Überschreiten eine Abweichung von dem Vergleichsmuster darstellt. Auf ähnliche Weise kann das wenigstens eine Vergleichsmuster eine wellenlängenabhängige Vergleichsintensität definieren, deren Unterschreiten eine Abweichung von dem Vergleichsmuster darstellt. Das Vergleichsmuster kann somit einen wellenlängenabhängigen Intensitätsbereich definieren, innerhalb dessen das Messspektrum als unauffällig eingestuft und keine Gasqualitätswarnung erzeugt wird.

Erfindungsgemäß wird das Messspektrum in Form eines Bildes dargestellt, welches abhängig von einer wellenlängenabhängigen Intensität in einen ersten Bereich und einen zweiten Bereich unterteilt ist. Die Abweichung wird durch Erfassen eines Überlapps des ersten Bereichs und/oder des zweiten Bereichs mit wenigstens einem von dem Vergleichsmuster definierten Vergleichsbereich erfasst. Dafür definiert das wenigstens eine Vergleichsmuster einen Vergleichsbereich, für welchen ein Überlapp mit dem ersten Bereich des Messspektrums eine Abweichung von dem Vergleichsmuster darstellt. Auf ähnliche Weise kann das wenigstens eine Vergleichsmuster einen Vergleichsbereich definieren, für welchen ein nicht vollständiger oder fehlender Überlapp mit dem ersten Bereich des Messspektrums eine Abweichung von dem Vergleichsmuster darstellt. Bei diesem Ausführungsbeispiel kann die Erfassung der Abweichung auf effiziente Weise unter Verwendung von Bildauswertungstechniken erfolgen. Gemäß einem Ausführungsbeispiel wird weiterhin eine Stärke der Abweichung erfasst und die Gasqualitätswarnung abhängig von der erfassten Stärke der Abweichung erzeugt. Beispielsweise kann die Stärke der Abweichung mit einem Schwellenwert verglichen werden und die Gasqualitätswarnung erzeugt werden, wenn die Stärke der Abweichung den Schwellenwert überschreitet. Es ist aber auch ein Vergleich der stärkere Abweichung mit mehreren Schwellenwerten möglich. So kann beispielsweise bei eine erste Stufe der Gasqualitätswarnung erzeugt werden, wenn die Stärke der Abweichung einen unteren Schwellenwert überschreitet, und eine zweite Stufe der Gasqualitätswarnung erzeugt werden, wenn die Stärke der Abweichung einen oberen Schwellenwert überschreitet.

Gemäß einem Ausführungsbeispiel wird abhängig von der erzeugten Gasqualitätswarnung eine Vorrichtung zur Beförderung von Gas in den zu überwachenden Raum und/oder aus dem zu überwachenden Raum gesteuert. Beispielweise kann die Vorrichtung eine Belüftungsanlage umfassen. Der zu überwachende Raum kann sich dann in einem Luftzufuhrabschnitt der Belüftungsanlage oder in einem über die Belüftungsanlage zu belüftenden Raum befinden. Gemäß einem weiteren Beispiel kann die Vorrichtung eine Prozessanlage umfassen. In diesem Fall kann der zu überwachende Raum sich in einem Zufuhrabschnitt für ein in der Prozessanlage verwendetes Prozessgas befinden oder sich in einem Prozessraum der Prozessanlage befinden.

Gemäß einem weiteren Ausführungsbeispiel der vorliegenden Erfindung wird eine Vorrichtung zur Gasqualitätsüberwachung bereitgestellt. Die Vorrichtung umfasst eine Spektrometereinrichtung zur spektroskopischen Untersuchung einer Gasprobe aus einem zu überwachenden Raum. Die spektroskopische Untersuchung liefert ein sich über einen Wellenlängenbereich erstreckendes Messspektrum. Weiterhin umfasst die Vorrichtung eine Auswerteeinrichtung. Die Auswerteeinrichtung ist dazu ausgestaltet ist eine Abweichung des Messspektrums von wenigstens einem Vergleichsmuster zu erfassen. Weiterhin ist die Auswerteeinrichtung dazu ausgestaltet, abhängig von der erfassten Abweichung eine Gasqualitätswarnung zu erzeugen. Die Vorrichtung zur Gasqualitätsüberwachung kann somit zur Durchführung des Verfahrens nach einem der obigen Ausführungsbeispiele ausgestaltet sein.

Gemäß einem weiteren Ausführungsbeispiel der vorliegenden Erfindung wird eine Belüftungsanlage bereitgestellt. Die Belüftungsanlage dient der Belüftung eines Raumes, z. B. ein Innenraum eines Fahrzeugs oder eines Gebäudes. Die Belüftungsanlage kann beispielsweise auch Teil eines Klimatisierungssystems sein. Die Belüftungsanlage umfasst eine Vorrichtung zur Beförderung von Luft in den zu belüftenden Raum und/oder aus dem zu belüftenden Raum. Weiterhin umfasst die Belüftungsanlage eine Vorrichtung zur Gasqualitätsüberwachung gemäß dem obigen Ausführungsbeispiel. Der zu überwachende Raum kann sich dabei in einem Luftzufuhrabschnitt der Belüftungsanlage oder in dem zu belüftenden Raum befinden. Weiterhin kann in diesem Fall die Vorrichtung zur Beförderung von Luft abhängig von der Gasqualitätswarnung gesteuert sein. So kann beispielweise abhängig von der Gasqualitätswarnung die Zufuhr von Luft in den zu belüftenden Raum unterbunden werden und/oder eine verstärkte Entlüftung des Raumes veranlasst werden.

Gemäß einem weiteren Ausführungsbeispiel der vorliegenden Erfindung wird eine Prozessanlage bereitgestellt. Die Prozessanlage umfasst eine Vorrichtung zur Beförderung von Gas in einen Prozessraum der Prozessanlage und/oder aus dem Prozessraum der Prozessanlage. Weiterhin umfasst die Prozessanlage eine Vorrichtung zur Gasqualitätsüberwachung gemäß dem obigen Ausführungsbeispiel. Der zu überwachende Raum sich in diesem Fall in einem Zufuhrabschnitt für ein in der Prozessanlage verwendetes Prozessgas befinden oder sich in dem Prozessraum der Prozessanlage befinden. Weiterhin kann auch in diesem Fall die Vorrichtung zur Beförderung von Gas abhängig von der Gasqualitätswarnung gesteuert sein. So kann beispielweise abhängig von der Gasqualitätswarnung die Zufuhr eines mangelhaften Prozessgases unterbunden werden und/oder eine verstärkte Abfuhr von Gas aus dem Prozessraum veranlasst werden. Weiterhin kann auch eine Gasreinigung veranlasst werden, z.B. durch Aktivieren einer Filtereinrichtung oder Einleiten eines chemischen Reinigungsprozesses.

Zum besseren Verständnis werden nachfolgend Ausführungsbeispiele unter Bezugnahme auf die beigefügten Zeichnungen näher erläutert.
Fig. 1 veranschaulicht schematisch eine Vorrichtung zur Gasqualitätsüberwachung gemäß einem Ausführungsbeispiel der Erfindung.
Fig. 2 zeigt ein Beispiel für ein Messspektrum, welches zur Gasqualitätsüberwachung gemäß einem Ausführungsbeispiel der Erfindung verwendet werden kann.
Fig. 3A und 3B veranschaulichen ein Beispiel für eine Auswertung von Abweichungen zwischen einem Messspektrum und einem Vergleichsmuster, das für sich genommen nicht unter den beanspruchten Gegenstand der Erfindung fällt.
Fig. 4A und 4B veranschaulichen ein weiteres Beispiel für eine Auswertung von Abweichungen zwischen einem Messspektrum und einem Vergleichsmuster, welches für sich genommen nicht unter den Gegenstand der beanspruchten Erfindung fällt.
Fig. 5A und 5B veranschaulichen ein weiteres Beispiel für eine Auswertung von Abweichungen zwischen einem Messspektrum und einem Vergleichsmuster gemäß einem Ausführungsbeispiel der Erfindung.
Fig. 6A und 6B veranschaulichen ein weiteres Beispiel für eine Auswertung von Abweichungen zwischen einem Messspektrum und einem Vergleichsmuster gemäß einem Ausführungsbeispiel der Erfindung.
Fig. 7 zeigt ein Flussdiagram zur Veranschaulichung eines Verfahrens zur Gasqualitätsüberwachung gemäß einem Ausführungsbeispiel der Erfindung.
Fig. 8 veranschaulicht ein Beispiel für ein Szenario, bei welchem eine Gasqualitätsüberwachung gemäß einem Ausführungsbeispiel der Erfindung in einer Belüftungsanlage verwendet wird.
Fig. 9 veranschaulicht ein Beispiel für ein Szenario, bei welchem eine Gasqualitätsüberwachung gemäß einem Ausführungsbeispiel der Erfindung in einer Prozessanlage verwendet wird.

Im Folgenden werden verschiedene Ausführungsbeispiele genauer erläutert. Diese detaillierte Beschreibung ist jedoch nicht als einschränkend auszulegen. Insbesondere ist eine Beschreibung eines Ausführungsbeispiels mit einer Vielzahl von Merkmalen, Komponenten oder Details nicht dahingehend auszulegen, dass alle diese Merkmale, Komponenten und Details zur Implementierung notwendig sind. Variationen und Abwandlungen, welche für eines der Ausführungsbeispiele beschrieben wurden, sind auch auf andere Ausführungsbeispiele anwendbar, sofern nichts anderes angegeben ist. Merkmale verschiedener Ausführungsbeispiele können zudem miteinander kombiniert werden, soweit die Kombinationen unter die Ansprüche fallen, um weitere Ausführungsbeispiele zu bilden.

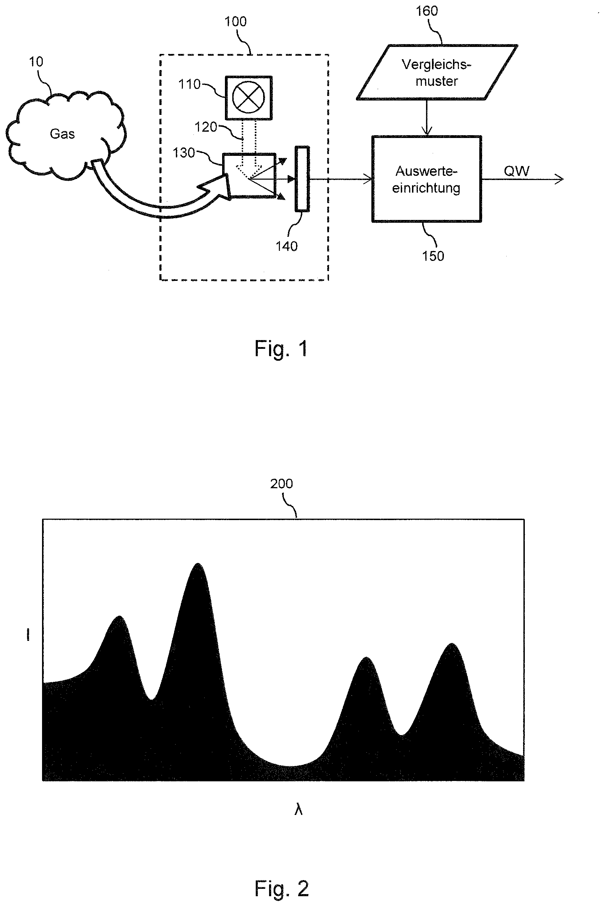

Die nachfolgend erläuterten Ausführungsbeispiele beziehen sich auf eine Gasqualitätsüberwachung im Zusammenhang mit einer Belüftungsanlage oder einer Prozessanlage. Fig. 1 veranschaulicht schematisch eine hierzu verwendete Vorrichtung zur Gasqualitätsüberwachung.

Wie in Fig. 1 dargestellt, weist die Vorrichtung eine Spektrometereinrichtung 100 und eine Auswerteinrichtung 150 auf. Die Spektrometereinrichtung dient der Untersuchung von Gasproben aus einem zu überwachenden Raum 10. Die zu untersuchenden Gasproben können in kurzen Zeitabständen, z.B. in der Größenordnung einer Sekunde oder weniger, entnommen werden, um so eine quasi-kontinuierliche Überwachung der Gasqualität zu ermöglichen. Bei dem zu überwachenden Raum 10 kann es sich beispielsweise um einen Innenraum eines Gebäudes oder eines Fahrzeugs handeln. Weiterhin kann es sich bei dem zu überwachenden Raum 10 um einen Luftzufuhrabschnitt oder einen Abluftabschnitt einer Belüftungsanlage für ein Gebäude oder ein Fahrzeug handeln. Weiterhin könnten die Gasproben auch im Freien entnommen werden. In diesen Anwendungsszenarien würde es sich bei einem in dem Raum 10 enthaltenen Gas um Luft handeln, d.h. die Vorrichtung würde zur Überwachung von Luftqualität eingesetzt werden. Bei anderen Anwendungsszenarien kann die Vorrichtung im Zusammenhang mit einer Prozessanlage verwendet werden. In diesem Fall kann es sich bei dem zu überwachenden Raum 10 um einen Prozessraum, z.B. um einen Brennraum oder eine Ätzkammer, um einen Teil einer Leitung für ein in der Prozessanlage verwendetes Prozessgas oder um einen Teil einer Abgasleitung handeln. Bei den letztgenannten Anwendungsszenarien würde es sich bei dem in dem Raum 10 enthaltenen Gas um ein Prozessgas oder Abgase eines Prozesses handeln.

Die Spektrometereinrichtung 100 ist bei dem dargestellten Beispiel als Raman-Spektrometer ausgestaltet und weist eine Strahlungsquelle 110 auf, welche Anregungslicht 120 erzeugt. Bei der Strahlungsquelle 110 kann es sich beispielsweise um einen Laser handeln. Weiterhin verfügt die Spektrometer Einrichtung über eine Messkammer 130, in welche die zu untersuchenden Gasproben eingeführt werden. Dies kann beispielsweise über ein in der Figur nicht dargestelltes Pumpsystem erfolgen. Das Anregungslicht 120 wird in die Messkammer 130 eingestrahlt, und in der Messkammer 130 gestreutes Licht wird von einem Detektor 140 erfasst, umso für jede Gasprobe ein Messspektrum zu gewinnen. Die Messspektren werden dann zur weiteren Analyse der Auswerteeinrichtung 150 zugeführt.

Die Auswerteeinrichtung 150 vergleicht die Messspektren mit einem Vergleichsmuster 160, um eine Abweichung des jeweiligen Messspektrums von dem Vergleichsmuster 160 festzustellen. Das Vergleichsmuster 160 basiert auf gemeinsamen Eigenschaften von Messspektren, welche zuvor als unauffällig eingestuft wurden. In einigen Fällen kann auch ein Vergleich des Messspektrums mit mehreren Vergleichsmustern 160 erfolgen. Wenn die Auswerteeinrichtung 150 eine Abweichung des Messspektrums von dem Vergleichsmuster 160 feststellt bzw. wenn die festgestellte Abweichung eine bestimmte Mindeststärke überschreitet, erzeugt die Auswerteeinrichtung 150 eine Gasqualitätswarnung QW. Bei der Gasqualitätswarnung QW kann es sich um ein Signal handeln, welches eine Gefahr mangelnder Gasqualität anzeigt. Die Gasqualitätswarnung QW kann beispielsweise als optisches oder akustisches Signal zur unmittelbaren Warnung von Personen ausgestaltet sein. Weiterhin kann es sich bei der Gasqualitätswarnung QW jedoch auch um ein elektronisches Signal handeln, durch welches automatisierte Steuervorgänge ausgelöst werden.

Um einen kompakten Aufbau der Spektrometereinrichtung 100 zu gewährleisten, können in dem Detektor 140 abbildende Beugungsgitter zum Einsatz kommen, welche in einem Bauelement sowohl eine spektralen Zerlegung des gestreuten Lichts als auch eine Abbildung auf eine Zeile oder ein Array von lichtempfindlichen Elementen bewerkstelligen. Diese abbildenden Beugungsgitter können zum Beispiel durch ein holografisches Verfahren mit einem Durchmesser von 10 mm oder weniger hergestellt werden und durch ein Spritzgussverfahren monolithisch in der Spektrometereinrichtung 100 integriert werden. Weiterhin könnte auch die Messkammer 130 mit einer Kavität zur Verstärkung des Anregungslichts 120 kombiniert werden. Durch eine oder mehrere dieser Maßnahmen kann ein kompakter Aufbau der Spektrometereinrichtung 100 mit einem Volumen von wenigen cm³ erreicht werden. Letzteres ist insbesondere bei einem Einsatz im Fahrzeugbereich von Vorteil. Eine kompakte Bauweise der Spektrometereinrichtung 100 kann jedoch auch in Belüftungsanlagen von Gebäuden oder in Prozessanlagen von Vorteil sein, weil hierdurch beispielsweise eine Anordnung der Spektrometereinrichtung 100 nahe am Ort der Entnahme der Gasprobe erleichtert wird. Auf diese Weise kann wiederum vermieden werden, dass Latenzzeiten und Totvolumina die Gasqualitätsüberwachung beeinträchtigen.

Fig. 2 zeigt ein beispielhaftes von dem Detektor 140 erfasstes Messspektrum 200. Wie in Fig. 2 dargestellt, zeigt das Messspektrum eine Intensität I des erfassten Streulichts in Abhängigkeit der Wellenlänge λ. Hierbei versteht es sich jedoch, dass die Abhängigkeit von der Wellenlänge λ auf äquivalente Weise auch als Abhängigkeit von Frequenz oder Wellenzahl dargestellt werden kann. Der hierin verwendete Begriff Anführungsstriche "wellenlängenabhängig" ist somit als gleichbedeutend mit "frequenzabhängig" oder "wellenzahlabhängig" verstehen. Ein von dem Messspektrum 200 abgedeckter Wellenlängenbereich kann dabei im Bereich von 100 nm bis 3000 nm liegen. Bei dem Messspektrum 200 kann es sich beispielsweise um ein Messspektrum im Ultraviolettbereich von ca. 180 nm bis ca. 400 nm, um ein Messspektrum im sichtbaren Bereich von ca. 380 nm bis ca. 800 nm, um ein Messspektrum im nahen Infrarotbereich von ca. 700 nm bis ca. 2500 nm handeln, um eine Kombination von zwei oder mehr dieser Bereiche, oder um Teile dieser Bereiche handeln.

Das in Fig. 2 beispielhaft dargestellte Messspektrum 200 zeigt keine scharfen Linien, sondern einen mehrere Banden aufweisenden Verlauf, welche zudem auch eine gewisse Untergrundintensität zeigt. Dieser Verlauf ist typisch für ein Szenario, bei welchem die untersuchte Gasprobe ein Gemisch verschiedener Gase ist und darüber hinaus auch flüssige oder feste Partikel enthalten kann. Eine Erkennung von einzelnen Bestandteilen dieses Gasgemisches anhand des Messspektrums ist in solchen Szenarien typischerweise äußerst komplex oder unter Umständen gar nicht möglich. Weiterhin kann auch bei einer bestimmten Zusammensetzung der Gasprobe das Messspektrum 200 eine erhebliche Variabilität in Abhängigkeit von Umgebungsparametern wie Temperatur, Druck oder Feuchtigkeitsgehalt aufweisen. Hierdurch wird eine Erkennung von einzelnen Bestandteilen des Gasgemisches zusätzlich erschwert. Die hierin beschriebene Gasqualitätsüberwachung beruht daher nicht auf einer Erkennung einzelner Bestandteile der untersuchten Gasprobe, sondern vielmehr auf der bereits erwähnten Erfassung von Abweichungen des Messspektrums 200 von einem oder mehreren Vergleichsmustern. Diese soll nachfolgend anhand von Beispielen näher erläutert werden.

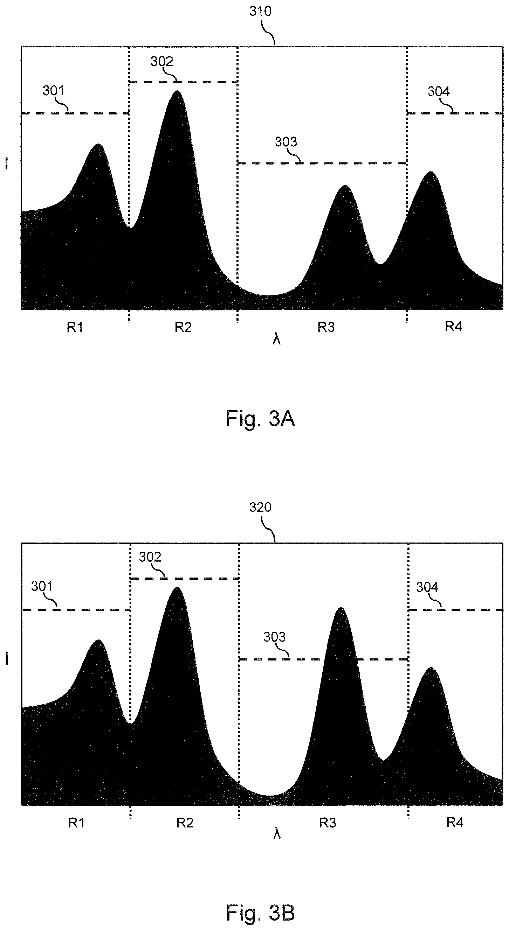

Fig. 3A und 3B fallen für sich genommen nicht unter den beanspruchten Gegenstand der Erfindung. Sie veranschaulichen ein Beispiel für eine Auswertung einer Abweichung zwischen einem Messspektrum 310, 320 und einem Vergleichsmuster, welches mehrere wellenlängenabhängige Vergleichsintensitäten 301, 302, 303, 304 definiert. Bei dem Beispiel von Fig. 3A und 3B beinhalten die Vergleichsintensitäten 301, 302, 303, 304 eine erste Vergleichsintensität 301 in einem ersten Wellenlängenbereich R1, eine zweite Vergleichsintensität 302 in einem zweiten Wellenlängenbereich R2, eine dritte Vergleichsintensität 303 in einem dritten Wellenlängenbereich R3 und eine vierte Vergleichsintensität 304 in einem vierten Wellenlängenbereich R4, welche sich voneinander unterscheiden. Bei diesem Beispiel entsprechen die Vergleichsintensitäten 301, 302, 303, 304 jeweils einer Maximalintensität, deren Überschreiten als Abweichung von dem Vergleichsmuster eingestuft wird. Eine entsprechende Maximalintensität kann beispielsweise durch Auswertung von als unauffällig eingestuften Messspektren bestimmt werden, indem der in dem jeweiligen Wellenlängenbereich R1, R2, R3, R4 maximal beobachtete Intensitätswert der als unauffällig eingestuften Messspektren ermittelt wird.

Bei dem in Fig. 3A dargestellten Szenario überschreiten die Intensitätswerte des Messspektrums 310 in keinem der Wellenlängenbereiche R1, R2, R3, R4 die Vergleichsintensitäten 301, 302, 303, 304. In diesem Fall wird somit keine Abweichung von dem Vergleichsmuster festgestellt und die Gasqualitätswarnung QW nicht erzeugt. Bei dem in Fig. 3B dargestellten Szenario überschreiten die Intensitätswerte des Messspektrums 320 jedoch in dem Wellenlängenbereiche R3 die entsprechende Vergleichsintensität 303. In diesem Fall wird somit eine Abweichung von dem Vergleichsmuster festgestellt und die Gasqualitätswarnung QW wird erzeugt.

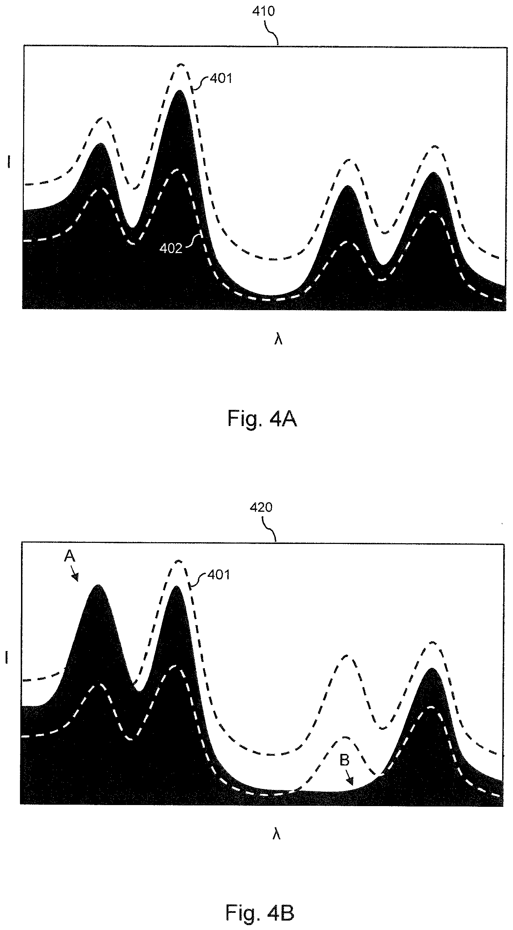

Fig. 4A und 4B fallen für sich genommen nicht unter den beanspruchten Gegenstand der Erfindung. Sie veranschaulichen ein weiteres Beispiel für eine Auswertung einer Abweichung zwischen einem Messspektrum 410, 420 und einem Vergleichsmuster, welches mehrere wellenlängenabhängige Vergleichsintensitäten 401, 402. Bei dem Beispiel von Fig. 4A und 4B beinhalten die Vergleichsintensitäten 401 einen ersten wellenlängenabhängigen Vergleichsintensitätsverlauf 401 und einen zweiten wellenlängenabhängigen Vergleichsintensitätsverlauf 402. Bei diesem Beispiel entspricht der erste Vergleichsintensitätsverlauf 401 einer wellenlängenabhängigen Maximalintensität, deren Überschreiten als Abweichung von dem Vergleichsmuster eingestuft wird. Der zweite Vergleichsintensitätsverlauf 402 entspricht einer wellenlängenabhängigen Minimalintensität, deren Unterschreiten als Abweichung von dem Vergleichsmuster eingestuft wird. Der wellenlängenabhängigen Vergleichsintensitätsverlauf 401 kann beispielsweise durch Auswertung von als unauffällig eingestuften Messspektren bestimmt werden, indem eine obere Einhüllende der als unauffällig eingestuften Messspektren ermittelt wird. Der wellenlängenabhängigen Vergleichsintensitätsverlauf 402 kann beispielsweise durch Auswertung von als unauffällig eingestuften Messspektren bestimmt werden, indem eine untere Einhüllende der als unauffällig eingestuften Messspektren ermittelt wird.

Bei dem in Fig. 4A dargestellten Szenario überschreiten die Intensitätswerte des Messspektrums 410 bei keiner Wellenlänge λ den Vergleichsintensitätsverlauf 401. Weiterhin unterschreiten die Intensitätswerte des Messspektrums 410 auch bei keiner Wellenlänge λ den Vergleichsintensitätsverlauf 402. In diesem Fall wird somit keine Abweichung von dem Vergleichsmuster festgestellt und die Gasqualitätswarnung QW nicht erzeugt. Bei dem in Fig. 4B dargestellten Szenario überschreiten die Intensitätswerte des Messspektrums 420 jedoch in einem mit A gekennzeichneten Bereich den Vergleichsintensitätsverlauf 401 und unterschreiten in einem mit B gekennzeichneten Bereich den Vergleichsintensitätsverlauf 402. In diesem Fall wird somit eine Abweichung von dem Vergleichsmuster festgestellt und die Gasqualitätswarnung QWwird erzeugt. Hierbei versteht es sich, dass die Überschreitung bei A und die Unterschreitung bei B jeweils einzeln als Abweichung aufgefasst werden können und auch unabhängig voneinander das erzeugen der Gasqualitätswarnung QW auslösen können. Bei dem Beispiel von Fig. 4A und 4B versteht es sich weiterhin, dass die wellenlängenabhängigen Vergleichsintensitätsverläufe 401 und 402 auch separat als Vergleichsmuster verwendet werden können.

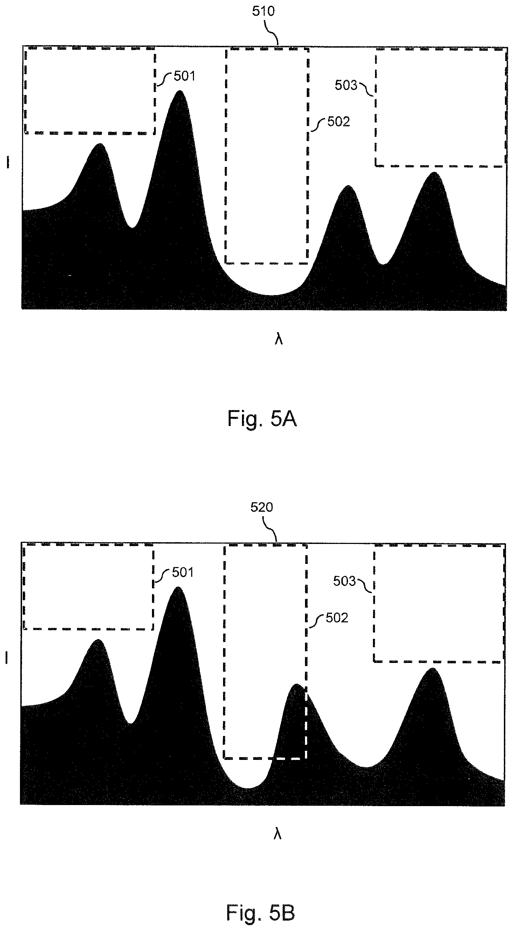

Fig. 5A und 5B veranschaulichen ein Beispiel für eine Auswertung einer Abweichung zwischen einem Messspektrum 510, 520 und einem Vergleichsmuster. Bei diesem Beispiel wird ausgenutzt das das Messspektrum 510, 520 als Bild dargestellt ist, welches durch den Intensitätsverlauf des Messspektrums 510, 520 in einen ersten Bereich und einen zweiten Bereich unterteilt ist. In Fig. 5A und 5B ist der erste Bereich als dunkle Fläche dargestellt. Der zweite Bereich ist als helle Fläche oberhalb der dunklen Fläche dargestellt. In diesem Fall kann der Vergleich des Messspektrums mit dem Vergleichsmuster anhand des Bildes mit den zwei Bereichen erfolgen. Hierbei können beispielsweise Bildanalysetechniken zum Einsatz kommen. Speziell definiert bei dem Beispiel von Fig. 5A und 5B das Vergleichsmuster Vergleichsbereiche 501, 502, 503. Ein Überlapp des dunkel dargestellten ersten Bereichs mit einem dieser Vergleichsbereiche 501, 502, 503 wird als Abweichung von dem Vergleichsmuster eingestuft. Die Vergleichsbereiche 501, 502, 503 können beispielsweise durch Auswertung von als unauffällig eingestuften Messspektren bestimmt werden, indem bei diesen Messspektren entsprechenden Bildern Bereiche erfasst werden, die bei allen Bildern Teil des hell dargestellten zweiten Bereichs sind. Hierbei versteht es sich, dass die Vergleichsbereiche 501, 502, 503 nicht unbedingt eine rechteckige Form aufweisen müssen, wie es in Fig. 5A und 5B dargestellt ist, sondern auch andere Formen der Vergleichsbereiche verwendet werden können, z.B. eine Dreieckform, unregelmäßige Polygone oder Formen mit kurvenförmiger Begrenzung. Weiterhin versteht es sich, dass auch für den hell dargestellten zweiten Bereich des Bildes ein oder mehrere Vergleichsbereiche definiert werden könnten, für welche erfasst wird, ob ein Überlapp mit dem zweiten Bereich des Bildes vorliegt.

Bei dem in Fig. 5A dargestellten Szenario besteht kein Überlapp des dunkel dargestellten ersten Bereichs mit einem der Vergleichsbereiche 501, 502, 503. In diesem Fall wird somit keine Abweichung von dem Vergleichsmuster festgestellt und die Gasqualitätswarnung QW nicht erzeugt. Bei dem in Fig. 5B dargestellten Szenario besteht ein Überlapp des dunkel dargestellten ersten Bereichs mit einem dem Vergleichsbereich 502. In diesem Fall wird somit eine Abweichung von dem Vergleichsmuster festgestellt und die Gasqualitätswarnung QW wird erzeugt.

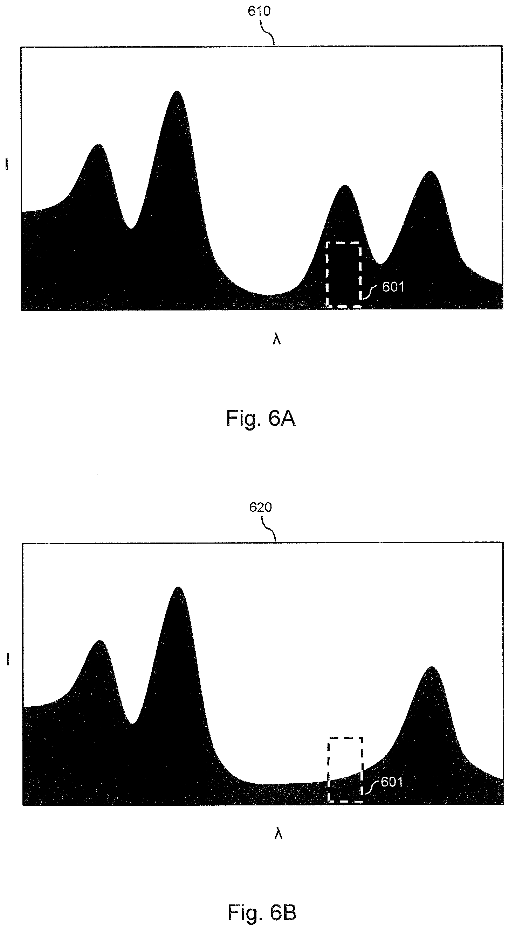

Fig. 6A und 6B veranschaulichen ein weiteres Beispiel für eine Auswertung einer Abweichung zwischen einem Messspektrum 610, 620 und einem Vergleichsmuster. Ähnlich wie bei dem Beispiel von Fig. 5A und 5B wird bei diesem Beispiel ausgenutzt das das Messspektrum 610, 620 als Bild dargestellt ist, welches durch den Intensitätsverlauf des Messspektrums 610, 620 in einen ersten Bereich und einen zweiten Bereich unterteilt ist. In Fig. 6A und 6B ist der erste Bereich als dunkle Fläche dargestellt. Der zweite Bereich ist als helle Fläche oberhalb der dunklen Fläche dargestellt. Ähnlich wie bei dem Beispiel von Fig. 5A und 5B kann auch in diesem Fall der Vergleich des Messspektrums mit dem Vergleichsmuster anhand des Bildes mit den zwei Bereichen erfolgen, z.B. unter Einsatz von Bildanalysetechniken. Bei dem Beispiel von Fig. 6A und 6B definiert das Vergleichsmuster einen Vergleichsbereich 601. Ein fehlender vollständiger Überlapp des dunkel dargestellten ersten Bereichs mit diesem Vergleichsbereich 601 wird als Abweichung von dem Vergleichsmuster eingestuft. Der Vergleichsbereich 601 kann beispielsweise durch Auswertung von als unauffällig eingestuften Messspektren bestimmt werden, indem bei diesen Messspektren entsprechenden Bildern Bereiche erfasst werden, die bei allen Bildern Teil des dunkel dargestellten ersten Bereichs sind. Auch bei diesem Beispiel versteht es sich, dass der Vergleichsbereich 601 nicht unbedingt eine rechteckige Form aufweisen müssen, wie es in Fig. 6A und 6B dargestellt ist, sondern auch andere Formen der verwendet werden können, z.B. eine Dreieckform, unregelmäßige Polygone oder Formen mit kurvenförmiger Begrenzung. Weiterhin versteht es sich, dass auch für den hell dargestellten zweiten Bereich des Bildes ein oder mehrere Vergleichsbereiche definiert werden könnten, für welche erfasst wird, ob ein Überlapp mit dem zweiten Bereich des Bildes vollständig ist.

Bei dem in Fig. 6A dargestellten Szenario besteht ein vollständiger Überlapp des dunkel dargestellten ersten Bereichs mit dem Vergleichsbereich 601. In diesem Fall wird somit keine Abweichung von dem Vergleichsmuster festgestellt und die Gasqualitätswarnung QW nicht erzeugt. Bei dem in Fig. 6B dargestellten Szenario ist der Überlapp des dunkel dargestellten ersten Bereichs mit dem Vergleichsbereich 601 unvollständig. In diesem Fall wird somit eine Abweichung von dem Vergleichsmuster festgestellt und die Gasqualitätswarnung QW wird erzeugt.

Es versteht sich, dass die anhand der oben genannten Beispiele beschriebenen Arten von Vergleichsmustern auch miteinander kombiniert werden können, soweit die Kombinationen unter die Ansprüche fallen. So könnten z.B. in manchen Wellenlängenbereichen wie anhand von Fig. 3A und 3B oder Fig. 4A und 4B erläutert Vergleichsintensitäten definiert werden und zusätzlich wie anhand von Fig. 5A und 5B oder Fig. 6A und 6B erläutert Vergleichsbereiche definiert werden.

Weiterhin kann bei den genannten Beispielen nicht nur das Vorhandensein einer Abweichung des Messspektrums von dem Vergleichsmuster erfasst werden, sondern auch eine Stärke der Abweichung, d.h. die Abweichung kann quantifiziert werden. Bei den Beispielen von Fig. 3A, 3B, 4A und 4B kann die Stärke beispielsweise auf Basis einer maximalen Differenz der Intensitätswerte des Messspektrums zu der Vergleichsintensität bestimmt werden. Bei den Beispielen von 5A, 5B, 6A und 6B kann die Stärke der Abweichung auf Basis eines Flächeninhalts des Vergleichsbereichs bestimmt werden, welcher von dem ersten Bereich des Messspektrums überlappt wird. In diesen Fällen kann das erzeugen der Gasqualitätswarnung QW abhängig von der Stärke der Abweichung erfolgen. Beispielsweise kann die Gasqualitätswarnung QW erzeugt werden, wenn die Stärke der Abweichung einen Schwellenwert überschreitet. Darüber hinaus kann die Gasqualitätswarnung QW auch mehrstufig erzeugt werden. So könnte beispielsweise eine erste Stufe der Gasqualitätswarnung QW erzeugt werden, wenn die Stärke der Abweichung einen ersten Schwellenwert überschreitet, und eine zweite Stufe der Gasqualitätswarnung QW erzeugt werden, wenn die Stärke der Abweichung einen zweiten Schwellenwert überschreitet. Abhängig von der mehrstufigen Gasqualitätswarnung QW können dann auch unterschiedliche automatisierte Steuervorgänge veranlasst werden.

Die verwendeten Vergleichsmuster können abhängig von der gewünschten Art der Gasqualitätsüberwachung ausgewählt werden. So kann beispielsweise eine Verunreinigung der Gasprobe auf effiziente Weise über Vergleichsmuster mit Maximalintensitäten, wie z.B. die Vergleichsintensitäten 301, 302, 303, 304 oder der Vergleichsintensitätsverlauf 401, oder über Vergleichsbereiche mit nicht gestattetem Überlapp, wie z.B. die Vergleichsbereiche 501, 502, 503, erkannt werden. Eine solche Verunreinigung kann zum Beispiel Schadstoffen oder unerwünschten Geruchsstoffen in einer Luftprobe entsprechen. Weiterhin kann eine unerwünschte Zusammensetzung von Bestandteilen der Gasprobe auf effiziente Weise über Vergleichsmuster mit Minimalintensitäten, wie z.B. der Vergleichsintensitätsverlauf 401, über Vergleichsmuster mit Maximalintensitäten, wie z.B. die Vergleichsintensitäten 301, 302, 303, 304 oder der Vergleichsintensitätsverlauf 401, über Vergleichsbereiche mit nicht gestattetem Überlapp, wie z.B. die Vergleichsbereiche 501, 502, 503, oder über Vergleichsbereiche mit erforderlichem Überlapp, wie z.B. der Vergleichsbereiche 601, erkannt werden. Eine solche unerwünschte Zusammensetzung von Bestandteilen der Gasprobe kann zum Beispiel einer zu geringen Konzentration eines Bestandteils eines Prozessgases oder einem zu geringen Sauerstoffanteil von Luft entsprechen.

Wie bereits erwähnt, besteht in vielen Anwendungsszenarien eine starke Abhängigkeit der erfassten Messspektren von Umgebungsparametern. So variieren im Falle einer Überwachung von Luft die Messspektren typischerweise in Abhängigkeit von Temperatur, Druck bzw. Luftdruck und Feuchtigkeitsgehalt bzw. Luftfeuchtigkeit in dem zu überwachenden Raum. Im Falle einer Überwachung eines Prozessgases kann wiederum eine Abhängigkeit der Messspektren von Temperatur oder Druck des Prozessgases bestehen. Diese Abhängigkeiten können bei der Auswertung der Messspektren berücksichtigt werden, indem einer oder mehrere der Umgebungsparameter erfasst werden und das zum Vergleich herangezogene Vergleichsmuster bzw. die zum Vergleich herangezogenen Vergleichsmuster abhängig von dem erfassten Umgebungsparameter ausgewählt werden. So können beispielsweise bei hoher Temperatur andere Vergleichsmuster genutzt werden als bei niedriger Temperatur. Die Erfassung des Umgebungsparameters bzw. der Umgebungsparameter kann über eine entsprechende Sensorik erfolgen, z.B. einen Temperatursensor, einen Drucksensor oder einen Luftfeuchtigkeitssensor.

Weiterhin können die Umgebungsparameter berücksichtigt werden, indem eine oder mehrere Referenzlinien in den Messspektren erfasst und vermessen werden. Solche Referenzlinien können gezielt über die Strahlungsquelle 110 der Spektrometereinrichtung 100 oder eine separate Referenzstrahlungsquelle erzeugt und/oder bezüglich Intensität oder Wellenlänge variiert werden. Aus einer bekannten Variation der Referenzlinie(n) in Abhängigkeit von dem bzw. den Umgebungsparametern kann dann auf den bzw. die Umgebungsparameter zurückgeschlossen werden. Weiterhin können die Referenzlinien auch genutzt werden, um eine Korrektur der erfassten Messspektren vorzunehmen, über welche durch variable Umgebungsparameter bedingte Einflüsse in den Messspektren kompensiert werden.

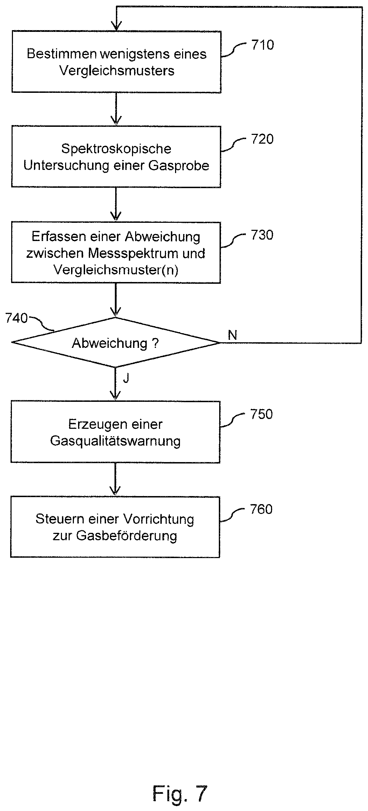

Fig. 7 zeigt ein Flussdiagram zur Veranschaulichung eines Verfahrens zur Gasqualitätsüberwachung gemäß einem Ausführungsbeispiel der Erfindung. Das Verfahren kann beispielsweise unter Verwendung der im Vorangegangenen erläuterten Vorrichtung zur Gasqualitätsüberwachung durchgeführt werden.

Bei Schritt 710 wird wenigstens ein Vergleichsmuster bestimmt. Das wenigstens eine Vergleichsmuster kann abhängig von einem oder mehreren Umgebungsparametern aus einer Vielzahl von Vergleichsmustern ausgewählt sein, z.B. abhängig von Temperatur, Druck, Luftfeuchtigkeit, oder dergleichen. Das wenigstens eine Vergleichsmuster kann beispielsweise anhand von als unauffällig eingestuften Messspektren bestimmt worden sein. Beispielsweise kann eine Vielzahl von Messspektren bei unterschiedlichen Umgebungsbedingungen erfasst werden und jeweils von einer Bedienperson als unauffällig eingestuft werden, wenn keine mangelnde Gasqualität der untersuchten Gasprobe vermutet wird. Zu diesem Zweck können auch zusätzliche Untersuchungen anderen analytischen Systemen vorgenommen werden. Hierbei kann auch ein maschinenbasierter Lernalgorithmus genutzt werden, z.B. auf Basis eines künstlichen neuronalen Netzwerks (KNN).

In manchen Szenarien können Vergleichsmuster jedoch auch im laufenden Betrieb angelernt werden. Zum Beispiel könnte wenn die Gasqualitätsüberwachung in Verbindung mit einer Belüftungsanlage für ein Fahrzeug durchgeführt wird eine Rückmeldung eines Fahrzeuginsassen, ob eine Geruchsbelästigung wahrgenommen wurde, erfasst werden und als Grundlage für die Einstufung eines zu diesem Zeitpunkt aufgenommenen Messspektrums als unauffällig herangezogen werden.

Bei Schritt 720 erfolgt eine spektroskopische Untersuchung einer Gasprobe aus einem zu überwachenden Raum. Die spektroskopische Untersuchung kann insbesondere durch Raman-Spektroskopie erfolgen. Bei dem zu überwachenden Raum kann es sich um einen Innenraum eines Fahrzeugs oder Gebäudes oder um einen Luftzufuhrabschnitt einer Belüftungsanlage handeln. In manchen Szenarien könnte die Gasprobe jedoch auch im Freien entnommen werden. Wenn die Gasqualitätsüberwachung im Zusammenhang mit einer Prozessanlage durchgeführt wird, kann es sich bei dem zu überwachenden Raum um einen Zufuhrabschnitt für ein Prozessgas, um einen Prozessraum der Prozessanlage, oder um einen Abgasabschnitt der Prozessanlage handeln.

Die spektroskopische Untersuchung der Gasprobe liefert ein Messspektrum, wie z.B. eines der oben genannten Messspektren 200, 310, 320, 410, 420, 510, 520, 610, 620. Das Messspektrum zeigt typischerweise einen wellenlängenabhängigen Intensitätsverlauf. Das Messspektrum wird als Bild dargestellt welches von dem wellenlängenabhängigen Intensitätsverlauf in einen ersten Bereich und einen zweiten Bereich unterteilt wird, wie es beispielsweise im Zusammenhang mit Fig. 5A, 5B, 6A und 6B erläutert wurde.

Bei Schritt 730 wird eine Abweichung zwischen dem Messspektrum und dem wenigstens einen Vergleichsmuster erfasst. Die Abweichung kann durch einen Vergleich einer wellenlängenabhängigen Intensität des Messspektrums mit wenigstens einer von dem Vergleichsmuster definierten Vergleichsintensität erfasst werden. Weiterhin wird wie bei Schritt 720 erwähnt das Messspektrum in Form eines Bildes dargestellt, welches abhängig von einer wellenlängenabhängigen Intensität in einen ersten Bereich und einen zweiten Bereich unterteilt ist. Die Abweichung erfolgt durch Erfassen eines Überlapps des ersten Bereichs und/oder des zweiten Bereichs mit wenigstens einem von dem Vergleichsmuster definierten Vergleichsbereich.

Weiterhin kann bei Schritt 730 auch eine Stärke der Abweichung zwischen dem Messspektrum und dem wenigstens einen Vergleichsmuster erfasst werden. Die Stärke kann beispielsweise durch einen numerischen Wert quantifiziert werden, welcher einen Vergleich mit einem oder mehreren Schwellenwerte ermöglicht.

Bei Schritt 740 erfolgt eine Überprüfung, ob bei Schritt 730 eine Abweichung erfasst wurde bzw. die bei Schritt 730 erfasste Abweichung eine bestimmte Mindeststärke aufweist. Letzteres kann beispielsweise durch Vergleich der Stärke der Abweichung mit einem Schwellenwert geschehen. Falls dies der Fall ist, wird das Verfahren mit Schritt 750 fortgesetzt, wie durch die Verzweigung "J" angedeutet. Andernfalls kann das Verfahren wie durch die Abzweigung "N" angedeutet zu Schritte 710 zurückkehren.

Bei Schritt 750 wird eine Gasqualitätswarnung erzeugt. Die Gasqualitätswarnung zeigt an, dass die Gefahr einer mangelnden Qualität eines Gases besteht, aus welchem die bei Schritt 720 untersuchten Gasprobe entnommen wurde. Die Gasqualitätswarnung kann als optisches und/oder akustisches Signal erzeugt werden. Weiterhin kann die Gasqualitätswarnung auch als elektronisches Signal erzeugt werden. Die Gasqualitätswarnung kann auch abhängig von einer bei Schritt 730 erfassten Stärke der Abweichung zwischen dem Messspektrum und dem wenigstens einen Vergleichsmuster erzeugt werden. Beispielsweise kann die Gasqualitätswarnung auf mehrstufige Weise erzeugt werden, indem die Stärke der Abweichung mit einem oder mehreren Schwellenwerten verglichen wird. So könnte eine erste Stufe der Gasqualitätswarnung erzeugt werden, wenn die Stärke der Abweichung einen ersten Schwellenwert überschreitet, und eine zweite Stufe der Gasqualitätswarnung erzeugt werden, wenn die Stärke der Abweichung einen höheren zweiten Schwellenwert überschreitet.

Bei Schritt 760 kann dann eine Vorrichtung zur Gasbeförderung abhängig von der bei Schritt 750 erzeugten Gasqualitätswarnung gesteuert werden. Beispielsweise kann die Gasbeförderung in Reaktion auf die Gasqualitätswarnung unterbunden werden, z.B. indem Ventile oder Klappen geschlossen werden und/oder eine Pumpe oder ein Gebläse deaktiviert wird. Weiterhin können in Reaktion auf die Gasqualitätswarnung Maßnahmen für eine Verbesserung der Gasqualität eingeleitet werden, z.B. indem eine Entlüftung des zu überwachenden Raums veranlasst wird.

Wie oben erwähnt, kann zum Anlernen der Vergleichsmuster ein maschinenbasierter Lernalgorithmus genutzt werden, z.B. auf Basis eines KNN. Der maschinenbasierte Lernalgorithmus kann anhand von vorab klassifizierten Messspektren und/oder im laufenden Betrieb trainiert werden. Im letztgenannten Fall kann beispielsweise eine Bedienperson Rückmeldungen an den Lernalgorithmus liefern, ob eine erzeugte Gasqualitätswarnung zutreffend war oder nicht. Weiterhin kann auch ein auf zusätzlicher Sensorik und/oder Analytik basierendes Trainings-Messsystem, welches problematische Gaszusammensetzungen gezielt identifiziert, zum Trainieren des maschinenbasierten Lernalgorithmus und Anlernen der Vergleichsmuster genutzt werden. Ein solches Trainings-Messsystem könnte lediglich zu Trainingszwecken genutzt werden, z.B. bei einer Erstinbetriebnahme, Kalibrierung oder anderweitigen Vorkonfigurierung der Vorrichtung zur Gasqualitätsüberwachung. Daher könnte das Trainings-Messsystem auf einer aufwändigeren Sensorik basieren als die Vorrichtung zur Gasqualitätsüberwachung. Weiterhin können beim Trainieren des maschinenbasierten Lernalgorithmus auch aufwändigere Analysen erfolgen, welche z.B. typischerweise nicht in Echtzeit vorgenommen werden können. Die Bestimmung der Vergleichsmuster kann durch den maschinenbasierten Lernalgorithmus vorab für eine Vielzahl von Vorrichtungen zur Gasqualitätsüberwachung vorgenommen werden, und die anhand von einer Vorrichtung gewonnenen Lernergebnisse können auf andere Vorrichtungen übertragen werden.

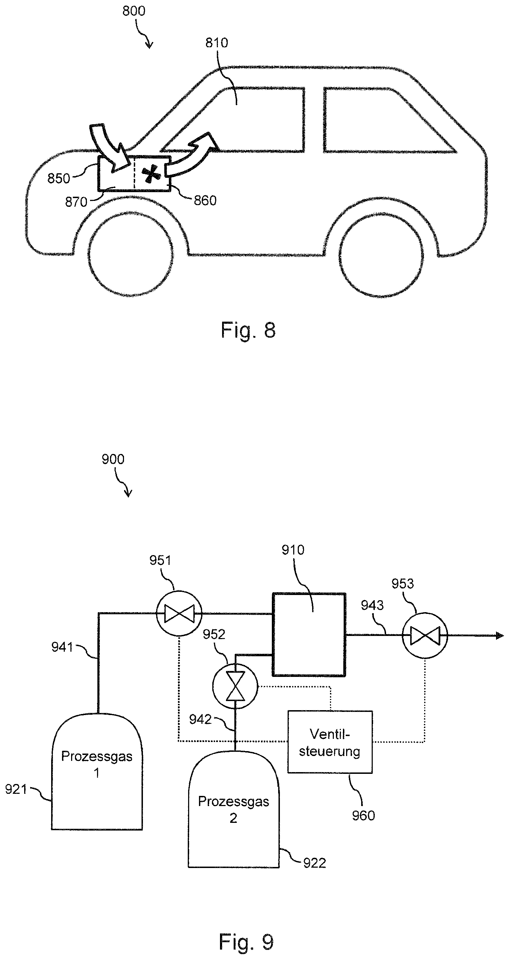

Fig. 8 veranschaulicht ein Beispiel für ein Szenario, bei welchem die oben beschriebene Gasqualitätsüberwachung gemäß in einer Belüftungsanlage verwendet wird. Speziell zeigt Fig. 8 ein Kraftfahrzeug 800, welches eine Belüftungsanlage 850 aufweist, die beispielsweise Teil eines Klimatisierungssystems sein kann. Hierbei versteht es sich jedoch, dass sich dieses Szenario auch ohne weiteres auf eine Belüftungsanlage in einer anderen Fahrzeugart, z.B. einem Flugzeug, einem Wasserfahrzeug oder einem Schienenfahrzeug, übertragen lässt.

Die Belüftungsanlage 850 verfügt über einen Luftzufuhrabschnitt 870, über welchen Luft von außen in die Belüftungsanlage 850 aufgenommen, und dann über ein Luftfördersystem 860 in einen Innenraum 810 des Kraftfahrzeugs 800 zugeführt wird. Das Luftfördersystem 860 kann beispielsweise auf einem Gebläse beruhen und darüber hinaus eine oder mehrere Klappen aufweisen, durch welche die Zufuhr von Luft von außen unterbunden werden kann. Letzteres ist beispielsweise wünschenswert, wenn in der Umgebung des Kraftfahrzeugs 800 unangenehme Gerüche auftreten oder das Kraftfahrzeug 800 sich in einem Tunnel oder einer Garage befindet.

In dem Szenario von Fig. 8 kann die Gasqualitätswarnung genutzt werden, um das Luftfördersystem 860 zu steuern. Wenn beispielsweise die Gasproben aus dem Luftzufuhrabschnitt 870 entnommen werden, können in Reaktion auf die Gasqualitätswarnung die Klappen des Luftfördersystems 860 geschlossen werden, um zu vermeiden dass Luft minderer Qualität in das Fahrzeuginnere 810 eingeleitet wird. Weiterhin können die Gasproben auch aus dem Innenraum 810 des Kraftfahrzeugs 800 entnommen werden. In diesem Fall kann in Reaktion auf die Gasqualitätswarnung veranlasst werden, dass das Luftfördersystem 860 verstärkt Luft von außen in den Innenraum 810 des Fahrzeugs 800 zuführt, um so die Qualität der Luft im Innenraum 810 zu verbessern. Weiterhin kann die Gasqualitätswarnung dem Fahrer oder anderen Insassen des Kraftfahrzeugs 800 auch in Form eines optischen oder akustischen Signals angezeigt werden. Darüber hinaus kann auch eine Luftreinigungsfunktion der Belüftungsanlage 850 aktiviert werden. Eine solche Luftreinigungsfunktion kann z.B. einer Filtereinrichtung basieren.

Fig. 9 veranschaulicht ein Beispiel für ein Szenario, die oben beschriebene Gasqualitätsüberwachung gemäß in einer Prozessanlage 900 verwendet wird. Die Prozessanlage kann beispielsweise der Herstellung oder Verarbeitung von Produkten dienen. Zum Beispiel könnten in der Prozessanlage Produkte mit einem oder mehreren Prozessgasen behandelt werden. Weiterhin könnte in der Prozessanlage auch eine chemische Reaktion von einem oder mehreren Prozessgasen erfolgen, z.B. Im Rahmen einer Verbrennung. Bei dem Beispiel von Fig. 9 weist die Prozessanlage 900 einen Prozessraum 910 auf, in welchen

Prozessgase aus einer ersten Gasquelle 921 und aus einer zweiten Gasquelle 922 zugeführt werden. Die Zuführung der Prozessgase erfolgt über Zufuhrleitungen 941, 942, welche mit steuerbaren Ventilen 951, 952 versehen sind. Weiterhin weist die Prozessanlage 900 auch eine Abgasleitung 943 auf, über welche bei den Prozessen in dem Prozessraum 910 entstehende Abgase abgeführt werden können. Auch die Abgasleitung 943 ist mit einem steuerbaren Ventil 953 versehen. Die Ventile 951,900 52,953 werden über eine Ventilsteuerung 960 gesteuert. Die Zufuhrleitungen 941,942, die Abgasleitung 943, die Ventile 951, 952,953, sowie die Ventilsteuerung 960 bilden ein Gasfördersystem der Prozessanlage 900.

In dem Szenario von Fig. 9 kann die Gasqualitätswarnung genutzt werden, um das Gasfördersystem der Prozessanlage 900 zu steuern. Wenn beispielsweise die Gasproben aus den Zufuhrleitungen 941 oder 942 entnommen werden, können in Reaktion auf die Gasqualitätswarnung die Ventile 951, 952 geschlossen werden, um zu vermeiden, dass Prozessgas minderer Qualität in den Prozessraum 910 eingeleitet wird. Weiterhin können auch Gasproben aus dem Prozessraum 910 entnommen werden. In diesem Fall kann in Reaktion auf die Gasqualitätswarnung veranlasst werden, dass das Ventil 943 geöffnet wird, um Gase minderer Qualität aus dem Prozessraum 910 zu entfernen. Darüber hinaus kann auch eine Gasreinigungsfunktion der Prozessanlage 900 aktiviert werden. Eine solche Gasreinigungsfunktion kann z.B. auf einer chemischen Reaktion in dem Prozessraum 910 basieren. Alternativ oder zusätzlich kann die Gasreinigung auch auf einer Filterung basieren. Weiterhin kann die Gasqualitätswarnung einer Bedienperson der Prozessanlage 900 in Form eines optischen oder akustischen Signals angezeigt werden oder als Auslöser für eine automatische Sicherheitsabschaltung der Prozessanlage 900 genutzt werden.

Es versteht sich, dass bei den im Vorangegangenen beschriebenen Ausführungsbeispielen verschiedene Modifikationen möglich sind, soweit sie unter die Ansprüche fallen. So ist die beschriebene Gasqualitätsüberwachung nicht auf Belüftungsanlagen oder Prozessanlagen beschränkt, sondern könnte auch in vielfältigen anderen Bereichen angewendet werden. Darüber hinaus können die hierin beschriebenen Vergleichsmuster auf verschiedene Weise modifiziert oder kombiniert werden, um so neue Arten von Vergleichsmustern zu generieren. Weiterhin könnten alternativ oder zusätzlich zur Gewinnung der Messspektren durch Raman-Spektroskopie auch andere spektroskopische Verfahren eingesetzt werden, z.B. Infrarotspektroskopie.

## Patentansprüche

1. Verfahren zur Gasqualitätsüberwachung, umfassend:
- spektroskopische Untersuchung einer Gasprobe aus einem zu überwachenden Raum, wobei die spektroskopische Untersuchung ein sich über einen Wellenlängenbereich erstreckendes Messspektrum (510, 520, 610, 620) liefert;
- Darstellen des Messspektrums (510, 520, 610, 620) in Form eines Bildes, welches in einen ersten Bereich und einen zweiten Bereich unterteilt ist, wobei die gemessene wellenlängenabhängige Intensität den ersten Bereich von dem zweiten Bereich trennt;
- Erfassen einer Abweichung des Messspektrums (510, 520, 610, 620) von wenigstens einem Vergleichsmuster (160) anhand des Bildes durch Erfassen eines Überlapps des ersten Bereichs und/oder des zweiten Bereichs mit wenigstens einem von dem Vergleichsmuster (160) definierten Vergleichsbereich (501, 502, 503; 601); und
- abhängig von der erfassten Abweichung, Erzeugen einer Gasqualitätswarnung (QW).

2. Verfahren nach Anspruch 1, umfassend:
- Erfassen wenigstens eines Umgebungsparameters des zu überwachenden Raums (10); und
- Auswählen des wenigstens einen Vergleichsmusters (160) abhängig von dem erfassten wenigstens einen Umgebungsparameter.

3. Verfahren nach Anspruch 2,
wobei der wenigstens eine Umgebungsparameter eine Temperatur, einen Druck und/oder einen Feuchtigkeitsgehalt umfasst.

4. Verfahren nach einem der vorhergehenden Ansprüche, umfassend:
- ein zusätzliches Erfassen der Abweichung durch einen Vergleich einer wellenlängenabhängigen Intensität des Messspektrums (310, 320; 410, 420) mit wenigstens einer von dem Vergleichsmuster (160) definierten Vergleichsintensität (301, 302, 303, 304; 401, 402).

5. Verfahren nach einem der vorhergehenden Ansprüche, umfassend:
- Erfassen einer Stärke der Abweichung; und
- Erzeugen der Gasqualitätswarnung (QW) abhängig von der erfassten Stärke der Abweichung.

6. Verfahren nach einem der vorhergehenden Ansprüche,
wobei das wenigstens eine Vergleichsmuster (160) gemeinsame Eigenschaften von als unauffällig eingestuften Messspektren beschreibt.

7. Verfahren nach einem der vorhergehenden Ansprüche,
wobei die spektroskopische Untersuchung der Gasprobe durch Raman-Spektroskopie erfolgt.

8. Verfahren nach einem der vorhergehenden Ansprüche, umfassend:
- Bestimmen des wenigstens einen Vergleichsmusters durch Trainieren eines maschinenbasierten Lernalgorithmus.

9. Verfahren nach einem der vorhergehenden Ansprüche, umfassend:
- abhängig von der erzeugten Gasqualitätswarnung (QW), Steuern einer Vorrichtung zur Beförderung von Gas in den zu überwachenden Raum und/oder aus dem zu überwachenden Raum (10).

10. Verfahren nach Anspruch 9,
wobei die Vorrichtung eine Belüftungsanlage (850) umfasst und der zu überwachende Raum sich in einem Luftzufuhrabschnitt (870) der Belüftungsanlage (850) oder in einem über die Belüftungsanlage (850) zu belüftenden Raum (810) befindet, oder
wobei die Vorrichtung eine Prozessanlage (900) umfasst und der zu überwachende Raum sich einem Zufuhrabschnitt (941, 942) für ein in der Prozessanlage (900) verwendetes Prozessgas befindet oder sich in einem Prozessraum (910) der Prozessanlage (900) befindet.

11. Vorrichtung zur Gasqualitätsüberwachung, umfassend:
eine Spektrometereinrichtung (100) zur spektroskopischen Untersuchung einer Gasprobe aus einem zu überwachenden Raum (10), wobei die spektroskopische Untersuchung ein sich über einen Wellenlängenbereich erstreckendes Messspektrum (510, 520; 610, 620) liefert; und
- eine Auswerteeinrichtung (150), welche dazu ausgestaltet ist:
- das Messspektrum (510, 520, 610, 620) in Form eines Bildes darzustellen, welches in einen ersten Bereich und einen zweiten Bereich unterteilt ist, wobei die gemessene wellenlängenabhängige Intensität den ersten Bereich von dem zweiten Bereich trennt;
- eine Abweichung des Messspektrums (510, 520, 610, 620) von wenigstens einem Vergleichsmuster (160) anhand des Bildes durch Erfassen eines Überlapps des ersten Bereichs und/oder des zweiten Bereichs mit wenigstens einem von dem Vergleichsmuster (160) definierten Vergleichsbereich (501, 502, 503; 601) zu erfassen; und
- abhängig von der erfassten Abweichung eine Gasqualitätswarnung (QW) zu erzeugen.

12. Vorrichtung nach Anspruch 11,
wobei die Vorrichtung zur Durchführung eines Verfahrens nach einem der Ansprüche 1 bis 10 ausgestaltet ist.

13. Belüftungsanlage (850), umfassend:
eine Vorrichtung (860) zur Beförderung von Luft in einen zu belüftenden Raum (810) und/oder aus dem zu belüftenden Raum (810); und
eine Vorrichtung zur Gasqualitätsüberwachung nach einem der Ansprüche 11 oder 12, wobei der zu überwachende Raum (810) sich in einem Luftzufuhrabschnitt (870) der Belüftungsanlage (850) oder in dem zu belüftenden Raum (810) befindet, und
die Vorrichtung (860) zur Beförderung von Luft abhängig von der Gasqualitätswarnung gesteuert ist.

14. Prozessanlage (900), umfassend:
eine Vorrichtung (941, 942, 943, 951, 952, 953, 960) zur Beförderung von Gas in einen Prozessraum (910) der Prozessanlage (900) und/oder aus dem Prozessraum (910) der Prozessanlage (900); und
eine Vorrichtung zur Gasqualitätsüberwachung nach Anspruch 11 oder 12,
wobei der zu überwachende Raum sich in einem Zufuhrabschnitt (941, 942) für ein in der Prozessanlage (900) verwendetes Prozessgas befindet oder sich in dem Prozessraum (910) der Prozessanlage (900) befindet, und
die Vorrichtung (941, 942, 943, 951, 952, 953, 960) zur Beförderung von Gas abhängig von der Gasqualitätswarnung gesteuert ist.

## Claims

1. A method for gas quality monitoring, comprising:
- spectroscopic analysis of a gas sample from a space to be monitored, wherein the spectroscopic analysis supplies a measurement spectrum (510, 520, 610, 620) extending over a wavelength range;
- representing the measurement spectrum (510, 520, 610, 620) in the form of an image, which is divided into a first area and a second area, wherein the measured wavelength-dependent intensity separates the first area from the second area;
- detecting a deviation of the measurement spectrum (510, 520, 610, 620) from at least one comparison pattern (160) on the basis of the image by detecting an overlap of the first area and/or the second area with at least one comparison area (501, 502, 503; 601) defined by the comparison pattern (160); and
- depending on the detected deviation, generating a gas quality warning (QW).

2. The method according to Claim 1, comprising:
- detecting at least one environmental parameter of the space (10) to be monitored; and
- selecting at least one comparison pattern (160) depending on the detected at least one environmental parameter.

3. The method according to Claim 2,
wherein the at least one environmental parameter comprises a temperature, a pressure, and/or a moisture content.

4. The method according to any one of the preceding claims, comprising:
- additionally detecting the deviation by a comparison of a wavelength-dependent intensity of the measurement spectrum (310, 320; 410, 420) to at least one comparison intensity (301, 302, 303, 304; 401, 402) defined by the comparison pattern (160).

5. The method according to any one of the preceding claims, comprising:
- detecting a strength of the deviation; and
- generating the gas quality warning (QW) depending on the detected strength of the deviation.

6. The method according to any one of the preceding claims,
wherein the at least one comparison pattern (160) describes common properties of measurement spectra classified as unremarkable.

7. The method according to any one of the preceding claims,
wherein the spectroscopic analysis of the gas sample is carried out by Raman spectroscopy.

8. The method according to any one of the preceding claims, comprising:
- determining the at least one comparison pattern by training a machine-based learning algorithm.

9. The method according to any one of the preceding claims, comprising:
- depending on the generated gas quality warning (QW), controlling a device for conveying gas into the space to be monitored or out of the space (10) to be monitored.

10. The method according to Claim 9,
wherein the device comprises a ventilation facility (850) and the space to be monitored is located in an air supply section (870) of the ventilation facility (850) or in a space (810) to be ventilated via the ventilation facility (850), or
wherein the device comprises a process facility (900) and the space to be monitored is located in a supply section (941, 942) for a process gas used in the process facility (900) or is located in a process space (910) of the process facility (900).

11. A device for gas quality monitoring, comprising:
a spectrometer unit (100) for the spectroscopic analysis of a gas sample from a space (10) to be monitored, wherein the spectroscopic analysis supplies a measurement spectrum (510, 520; 610, 620) extending over a wavelength range; and
- an evaluation unit (150), which is designed:
- to represent the measurement spectrum (510, 520, 610, 620) in the form of an image, which is divided into a first area and a second area, wherein the measured wavelength-dependent intensity separates the first area from the second area;
- to detect a deviation of the measurement spectrum (510, 520, 610, 620) from at least one comparison pattern (160) on the basis of the image by detecting an overlap of the first area and/or the second area with at least one comparison area (501, 502, 503; 601) defined by the comparison pattern (160); and
- to generate a gas quality warning (QW) depending on the detected deviation.

12. The device according to Claim 11,
wherein the device is designed to carry out a method according to any one of Claims 1 to 10.

13. A ventilation facility (850), comprising:
- a device (860) for conveying air into a space (810) to be ventilated and/or out of the space (810) to be ventilated; and
a device for gas quality monitoring according to one of Claims 11 or 12, wherein the space (810) to be monitored is located in an air supply section (870) of the ventilation facility (850) or in the space (810) to be ventilated, and
the device (860) for conveying air is controlled depending on the gas quality warning.

14. A process facility (900), comprising:
a device (941, 942, 943, 951, 952, 953, 960) for conveying gas into a process space (910) of the process facility (900) and/or out of the process space (910) of the process facility (900); and
a device for gas quality monitoring according to Claim 11 or 12,
wherein the space to be monitored is located in a supply section (941, 942) for a process gas used in the process facility (900) or is located in the process space (910) of the process facility (900), and
the device (941, 942, 943, 951, 952, 953, 960) for conveying gas is controlled depending on the gas quality warning.

## Revendications

1. Procédé de surveillance de la qualité d'un gaz, ledit procédé comprenant les étapes suivantes :
- effectuer une analyse spectroscopique d'un échantillon de gaz provenant d'un espace à surveiller, l'analyse spectroscopique fournissant un spectre de mesure (510, 520, 610, 620) qui s'étend dans une gamme de longueurs d'onde ;
- représenter le spectre de mesure (510, 520, 610, 620) sous la forme d'une image qui est divisée en une première zone et une deuxième zone, l'intensité mesurée dépendant de la longueur d'onde séparant la première zone de la deuxième zone ;
- détecter un écart entre le spectre de mesure (510, 520, 610, 620) et au moins un modèle de comparaison (160) sur la base de l'image par détection d'un chevauchement de la première zone et/ou de la deuxième zone avec au moins une zone de comparaison (501, 502, 503 ; 601) défini par le modèle de comparaison (160) ; et
- générer un avertissement de qualité de gaz (GW) en fonction de l'écart détecté.

2. Procédé selon la revendication 1, comprenant :
- détecter au moins un paramètre d'environnement de l'espace à surveiller (10) ; et
- sélectionner l'au moins un modèle de comparaison (160) en fonction de l'au moins un paramètre d'environnement détecté.

3. Procédé selon la revendication 2,
l'au moins un paramètre d'environnement comprenant une température, une pression et/ou une teneur en humidité.

4. Procédé selon l'une des revendications précédentes, comprenant les étapes suivantes :
- effectuer une détection supplémentaire de l'écart par comparaison d'une intensité dépendante de la longueur d'onde du spectre de mesure (310, 320 ; 410, 420) à au moins une intensité de comparaison (301, 302, 303, 304 ; 401, 402) définie par le modèle de comparaison (160).

5. Procédé selon l'une des revendications précédentes, comprenant les étapes suivantes :
- détecter une ampleur de l'écart ; et
- générer l'avertissement de qualité de gaz (QW) en fonction de l'ampleur de l'écart détectée.

6. Procédé selon l'une des revendications précédentes,
l'au moins un modèle de comparaison (160) décrivant des propriétés communes de spectres de mesure classés comme non suspects.

7. Procédé selon l'une des revendications précédentes,
l'analyse spectroscopique de l'échantillon de gaz étant effectuée par spectroscopie Raman.

8. Procédé selon l'une des revendications précédentes, comprenant les étapes suivantes :
- déterminer l'au moins un modèle de comparaison par entraînement d'un algorithme d'apprentissage par machine.

9. Procédé selon l'une des revendications précédentes, comprenant l'étape suivante :
- en fonction de l'avertissement de qualité de gaz (QW) généré, commander un dispositif de transport de gaz jusque dans l'espace à surveiller (10) et/ou depuis celui-ci.

10. Procédé selon la revendication 9,
le dispositif comprenant une installation de ventilation (850) et l'espace à surveiller étant situé dans une portion d'alimentation en air (870) de l'installation de ventilation (850) ou dans un espace (810) à ventiler par le biais de l'installation de ventilation (850), ou
le dispositif comprenant une installation de traitement (900) et l'espace à surveiller étant situé dans une portion d'alimentation (941, 942) destinée à un gaz de traitement utilisé dans l'installation de traitement (900) ou étant situé dans un espace de traitement (910) de l'installation de traitement (900).

11. Dispositif de surveillance de la qualité d'un gaz, ledit dispositif comprenant :
un module de spectrométrie (100) destiné à effectuer l'analyse spectroscopique d'un échantillon de gaz provenant d'un espace à surveiller (10), l'analyse spectroscopique fournissant un spectre de mesure (510, 520 ; 610, 620) qui s'étend dans une gamme de longueurs d'onde ; et
- un module d'évaluation (150) qui est conçu pour :
- représenter le spectre de mesure (510, 520, 610, 620) sous la forme d'une image qui est divisée en une première zone et une deuxième zone, l'intensité mesurée dépendant de la longueur d'onde séparant la première zone de la deuxième zone ;
- détecter un écart entre le spectre de mesure (510, 520, 610, 620) et l'au moins un modèle de comparaison (160) sur la base de l'image par détection d'un chevauchement de la première zone et/ou de la deuxième zone avec au moins une zone de comparaison (501, 502, 503 ; 601) définie par le modèle de comparaison (160) ; et
- générer un avertissement de qualité de gaz (QW) en fonction de l'écart détecté.

12. Dispositif selon la revendication 11,
le dispositif étant conçu pour mettre en œuvre un procédé selon l'une des revendications 1 à 10.

13. Installation de ventilation (850) comprenant :
un dispositif (860) destiné à transporter de l'air jusque dans un espace à ventiler (810) et/ou depuis l'espace à ventiler (810) ; et
un dispositif de surveillance de la qualité d'un gaz selon l'une des revendications 11 et 12,
l'espace à surveiller (810) étant situé dans une portion d'alimentation en air (870) de l'installation de ventilation (850) ou dans l'espace à ventiler(810), et
le dispositif (860) destiné à transporter de l'air étant commandé en fonction de l'avertissement de qualité de gaz.

14. Installation de traitement (900) comprenant :
un dispositif (941, 942, 943, 951, 952, 953, 960) destiné à transporter du gaz jusque dans un espace de traitement (910) de l'installation de traitement (900) et/ou depuis l'espace de traitement (910) de l'installation de traitement (900) ; et
un dispositif de surveillance de la qualité d'un gaz selon la revendication 11 ou 12,
l'espace à surveiller étant situé dans une portion d'alimentation (941, 942) destinée à un gaz de traitement utilisé dans l'installation de traitement (900) ou étant situé dans l'espace de traitement (910) de l'installation de traitement (900), et
le dispositif (941, 942, 943, 951, 952, 953, 960) étant commandé pour transporter un gaz en fonction de l'avertissement de qualité de gaz.
